# EUROPÄISCHE PATENTANMELDUNG

(11) **EP 2 557 313 A1**
(43) Veröffentlichungstag der Anmeldung: **13.02.2013**
(21) Anmeldenummer: 11075191.4
(22) Anmeldetag: 10.08.2011
(51) Int. Cl.: F04D 3/02, F04D 13/06, F04D 29/18, A61M 1/10

(54) **Rotationspumpe mit einem rotor und förderelementen**

(71) Anmelder: Berlin Heart GmbH, 12247 Berlin (DE)
(72) Erfinder: Graichen, Kurt, Dr., 13189 Berlin (DE)
(74) Vertreter: Pfenning, Meinig & Partner GbR

(57) **Zusammenfassung**

Die Erfindung bezieht sich auf eine Rotationspumpe mit einem Rotor (1, 1', 1"), der Förderelemente (3, 3 ' , 3'', 4, 4', 4'') aufweist, welche ein Fluid wenigstens teilweise in Axialrichtung des Rotors fördern. Zur Förderung des Fluids sind an dem Rotor zwei Förderelemente bzw. Gruppen von Förderelementen vorgesehen, die das zu fördernde Fluid in einander entgegengesetzten Axialrichtungen des Rotors fördern, so dass sich die Axialschubkraftkomponenten weitgehend kompensieren. Die gegeneinander laufenden Fluidströmungen weichen gemeinsam in radialer Richtung des Rotor aus und sind durch ein Spiralgehäuse (9) gemeinsam ableitbar und nutzbar.

## Beschreibung

Die Erfindung liegt auf dem Gebiet der Mechanik, insbesondere der Pumpentechnik, und befasst sich mit Rotationspumpen.

Derartige Rotationspumpen sind in Form von Axialpumpen und Radialpumpen bekannt, jeweils mit Rotoren, die Förderelemente, beispielsweise in Form von Schaufeln, aufweisen. Je nach der geometrischen Form der Förderelemente/Schaufeln findet eine Förderung eines Fluids vorwiegend in Axialrichtung, in Radialrichtung oder in gemischten Richtungen statt.

Derartige Rotationspumpen können mit Vorteil beispielsweise in der Medizintechnik zur Förderung von körpereigenen Flüssigkeiten, beispielsweise als Blutpumpen, eingesetzt werden.

Insbesondere bei Axialpumpen stellt sich dabei auch die Frage nach der Qualität und Art der Lagerung. Da solche Pumpen oft sehr schnell laufen, beispielsweise mit bis zu 10.000 oder 20.000 U/min, und dabei nur ein Minimum von Abrieb verursacht werden soll und da außerdem die Wärmeentwicklung, der Verschleiß und auch der Energieverbrauch in einer solchen Pumpe minimiert werden soll, werden an die Lagerung erhöhte Anforderungen gestellt.

Dabei ist es insbesondere für die Axiallagerung schwierig, eine einfache und kostengünstige Lösung zu schaffen.

Die vorliegende Erfindung hat sich daher die Aufgabe gestellt, eine Rotationspumpe der eingangs genannten Art zu schaffen, die bei einfacher Bauart möglichst geringe Anforderungen an die Lagerung stellt.

Die Aufgabe wird mit den Merkmalen der Erfindung gemäß Patentanspruch 1 gelöst.

Dabei sind einerseits Förderelemente auf dem Rotor der Rotationspumpe vorgesehen, die das zu fördernde Fluid in Axialrichtung des Rotors in einem ersten Richtungssinn fördern, andererseits sind andere Förderelemente auf dem Rotor vorgesehen, die das Fluid ebenfalls in Axialrichtung, aber im entgegengesetzten Richtungssinn fördern.

Dadurch wirken die jeweils erzeugten Axialschubkräfte beider gegensinnig wirkender Förderelemente oder Gruppen von Förderelementen ebenfalls gegensinnig und heben sich im Idealfall auf. Es ergibt sich ein minimierter resultierender Axialschub der Pumpe trotz axialer Förderung eines Fluids.

Vorteilhaft soll dabei der Rotordurchmesser und/oder die aktive, fördernde Querschnittsfläche der Förderelemente, in Strömungsrichtung gesehen, für die einen und die anderen Förderelemente gleich sein, insbesondere um weniger als 50% der jeweils größeren Fläche, vorzugsweise nicht mehr als 10%, differieren.

Es ist zudem vorteilhaft, wenn auch die beiden Fluidströme sich um nicht mehr als 50% des größeren Fluidstroms, vorzugsweise um weniger als 30%, weiter vorzugsweise weniger als 20%, insbesondere weniger als 10%, unterscheiden.

Auch die Nabe kann im Bereich der einen und der anderen Förderelemente im Wesentlichen denselben Durchmesser haben, wobei die Unterschiede geringer als 20% des größeren Durchmessers, insbesondere geringer als 5% des größeren Durchmessers, sein sollten.

Grundsätzlich ist zudem natürlich sicherzustellen, dass die gegensinnigen Fluidströme derart gelenkt werden, dass ein resultierender Gesamtstrom des Fluids entsteht, der der gewünschten Förderung entspricht. Diese Lenkung bzw. Leitung der Fluidströme kann jedoch ohne Erzeugung zusätzlicher auf den Rotor wirkender Axialkräfte geschehen, wie anhand von Ausführungsbeispielen der Erfindung im Folgenden erläutert wird.

Insbesondere kann vorteilhaft vorgesehen sein, dass der durch das erste Förderelement oder eine erste Gruppe von Förderelementen geförderte erste Fluidstrom und der durch das zweite Förderelement oder eine zweite Gruppe von Förderelementen geförderte zweite Fluidstrom voneinander separiert sind.

Dabei kann mit Vorteil vorgesehen sein, dass der erste und der zweite Fluidstrom vor der Berührung mit dem ersten und dem weiteren Förderelement oder ersten und zweiten Gruppen von Förderelementen voneinander separiert werden.

Dies kann beispielsweise dadurch realisiert werden, dass das erste Förderelement oder die erste Gruppe von Förderelementen und das oder die weiteren Förderelemente aus unterschiedlichen Bereichen eines Fluidreservoirs das Fluid ansaugen und dieses getrennt voneinander fördern. Die auf diese Weise separierten Fluidströme können dann nach der Förderung durch die jeweiligen Förderelemente geeignet zusammengeführt werden.

Eine vorteilhafte Realisierungsform der Erfindung sieht daher vor, dass der erste und der zweite Fluidstrom nach dem Durchlaufen des ersten und des weiteren Förderelementes oder einer ersten und zweiten Gruppe von Förderelementen vereinigt werden.

Dabei kann beispielsweise vorgesehen sein, dass das erste und das zweite Förderelement oder die erste und zweite Gruppe von Förderelementen den ersten und den zweiten Fluidstrom aufeinander zugerichtet fördern und dass der erste und der zweite Fluidstrom gemeinsam radial nach außen abgelenkt werden.

Beispielsweise können das erste Förderelement oder eine erste Gruppe von Förderelementen durch Förderschaufeln oder eine einzelne, schraubenartig um eine Rotornabe umlaufend angeordnete Förderschaufel gebildet sein, die einzeln für sich oder in der Zusammenwirkung eine axiale Förderung des Fluids bewirken. Das oder die weiteren Förderelemente können ebenso ausgestaltet sein wie das oder die ersten Förderelemente, jedoch mit entsprechend umgekehrter Anordnung, so dass die Förderung in eine axial entgegengesetzte Richtung stattfindet. Idealerweise sollte der jeweils erzeugte Axialschub der verschiedenen Gruppen von Förderelementen vom Betrag her gleich groß sein. Dies kann dadurch erreicht werden, dass die Förderelemente umgekehrt gleich ausgebildet sind oder dass beispielsweise die ersten Förderelemente einen anderen Anstellwinkel, dafür jedoch eine entsprechend andere, angepasste Fläche oder Verteilung über die axiale Länge des Rotors aufweisen als die zweite Gruppe von Förderelementen. Die Art der Erzeugung des entsprechenden Axialschubs kann somit durch unterschiedliche geometrische Gestaltung der Förderelemente unterschiedlich sein, wobei naturgemäß die Drehzahl aller Förderelemente wegen der Anordnung auf einem gemeinsamen Rotor gleich ist und wobei zudem auch der erzeugte Axialschub möglichst betragsmäßig gleich sein sollte.

Werden die durch die verschiedenen Gruppen von Förderelementen erzeugten gegensinnigen und aufeinander zulaufenden Fluidströme am Umfang des Rotors gegeneinander geleitet, so ergibt sich zwangsweise eine radiale Ablenkung, beispielsweise nach außen oder in Umfangsrichtung. Zwischen den Teilströmen wird hierbei bei der Vermischung eine Trennfläche gebildet, entlang derer die Teilströmungen parallel zueinander laufen und sich gegebenenfalls vermischen.

Zusätzlich kann zur Umlenkung der Fluidströmungen von der Axialrichtung in die Radialrichtung vorteilhaft eine Leiteinrichtung auf dem Rotor befestigt sein. Diese kann durch eine ringförmig am Rotor umlaufende Erhebung mit beiderseits konkav ausgebildeter schräger Anlauffläche gebildet sein. Es kann jedoch auch einfach ein im Radialschnitt dreieckig ausgebildeter Kreisring auf dem Rotor vorgesehen sein.

Vorteilhaft ist zur Ableitung des Fluidstromes vorgesehen, dass das erste und das weitere Förderelement in verschiedenen Abschnitten des Rotors angeordnet sind und dass axial zwischen diesen Abschnitten ein Spiralgehäuse (Auslassgehäuse) vorgesehen ist, das den Rotor umgibt. Ein derartiges Spiralgehäuse kann einfach durch eine umfangsseitige Erweiterung des Pumpengehäuses mit einem integrierten radialen Auslass gebildet sein. Die umfangsseitige Mantelfläche dieser Erweiterung kann auch die geometrische Form einer Spirale haben, um die Ausleitung des Fluids zu begünstigen.

Eine besonders vorteilhafte Ausgestaltung der Erfindung sieht vor, dass innerhalb des Pumpengehäuses mehrere separierte Fluidkanäle vorgesehen sind, die einen Fluidraum mit verschiedenen Axialabschnitten, insbesondere den Enden, des Rotors verbinden. Hierdurch können die verschiedenen Axialabschnitte des Rotors, an denen gegensinnig fördernde Förderelemente angeordnet sind, jeweils mit separat zuströmenden Fluidströmen versorgt werden.

Ebenso kann auch vorgesehen sein, dass der Rotor mit den verschiedenen Gruppen von Förderelementen zwei Fluidströme axial jeweils zu den verschiedenen Enden des Rotors fördert und insgesamt das Fluid in radialer Richtung im axial mittleren Bereich des Rotors ansaugt. In diesem Fall können die separaten Fluidströme über die verschiedenen Fluidkanäle abströmen und in einem Fluidraum vereinigt werden.

Besonders vorteilhaft kann vorgesehen sein, dass die Nabe des Rotors hohl ausgebildet ist und einen Fluidkanal bildet. Der Rotor kann in diesem Fall an einem seiner stirnseitigen Enden das Fluid ansaugen, wobei ein erster Teil des Fluidstroms im radial äußeren Bereich des Rotors entlang strömt und ein zweiter Teil durch das Innere der Rotornabe zum gegenüberliegenden Ende des Rotors. Dort, an dem gegenüberliegenden Ende des Rotors, trifft dieser Fluidstrom dann nach einer Umlenkung auf die weiteren Förderelemente, die eine gegensinnige Förderung in Axialrichtung bewirken und den dort geförderten Fluidstrom gegensinnig gegen den erstgenannten Fluidstrom fördern. Die Umlenkung kann durch geeignete Formgebung des Umlenkelements auch die Zuströmung zu den Förderelementen durch Erzeugung eines Vordralls geeignet beeinflussen.

Dabei kann vorgesehen sein, dass an feststehenden Teilen der Rotorpumpe feststehende Lenkungseinrichtungen für Fluidströme vorgesehen sind. Beispielsweise kann konkret vorgesehen sein, dass in axialer Verlängerung der Rotornabe, sofern diese hohl ausgebildet ist, eine Pralleinrichtung als Lenkungseinrichtung zur Umlenkung eines Fluidstroms aus einer Axialrichtung in eine oder mehrere Radialrichtungen, insbesondere mit einer zentralen Erhebung, angeordnet ist. Die zentrale Erhebung kann beispielsweise ein Kegel oder eine kegelähnliche Form, vorteilhaft mit konkav ausgebildeten Flanken, sein.

Als weitere Lenkungseinrichtungen für die Fluidströme können einseitig oder beidseitig in Verlängerung des Rotors insbesondere feststehende Leitschaufeln vorgesehen sein, die entweder gerade ausgebildete Leitschaufeln oder schraubenartig angeordnete Leitschaufeln sein können, wodurch der insgesamt schraubenartig gerichtete Fluidstrom entlang des Rotors optimiert werden kann. So kann ein geeigneter Vordrall beim Zustrom auf die Förderelemente erzeugt werden.

Durch die beschriebene Erzeugung und Ausrichtung bzw. Lenkung von Fluidströmen kann in jedem Fall der auf einen Rotor wirkende resultierende Gesamtaxialschub verringert werden, so dass gegenüber bisher üblichen Lösungen weniger aufwendige Axiallagergestaltungen gewählt werden können. Beispielsweise können aktive oder passive axiale Magnetlager oder auch geregelte Magnetlager verwendet werden. Grundsätzlich können auch die magnetischen Elemente eines elektromotorischen Antriebs eines Rotors bereits für die Gestaltung eines axialen Magnetlagers genutzt werden. Diese können für sich allein vorteilhaft schon ausreichen.

Im Folgenden wird die Erfindung anhand eines Ausführungsbeispiels in einer Zeichnung gezeigt und anschließend erläutert. Dabei zeigt
- Fig. 1: schematisch einen Längsschnitt durch eine Rotationspumpe mit einem hohl ausgebildeten Rotor,
- Fig. 2: eine dreidimensionale Ansicht einer Rotationspumpe mit einem durchbrochenen Gehäuse,
- Fig. 3: einen Längsschnitt durch eine Rotationspumpe mit einem hohlen Rotor und einer Lagereinrichtung,
- Fig. 4: einen Längsschnitt durch eine Rotationspumpe mit einem Rotor, der Stützringe zur Lagerung aufweist, sowie mit einem Magnetlager,
- Fig. 5: einen Längsschnitt durch eine Rotationspumpe ähnlich der aus Fig. 4 mit mehreren Magnetlagereinrichtungen,
- Fig. 6: einen Längsschnitt durch eine Rotationspumpe mit einem hohlen Rotor, einer Lagereinrichtung sowie stehenden Leitschaufeln sowie
- Fig. 7: einen weiteren Längsschnitt durch eine Rotationspumpe mit einem hohlen Rotor und einer axial zu diesem angeordneten aktiven Magnetlagereinrichtung.

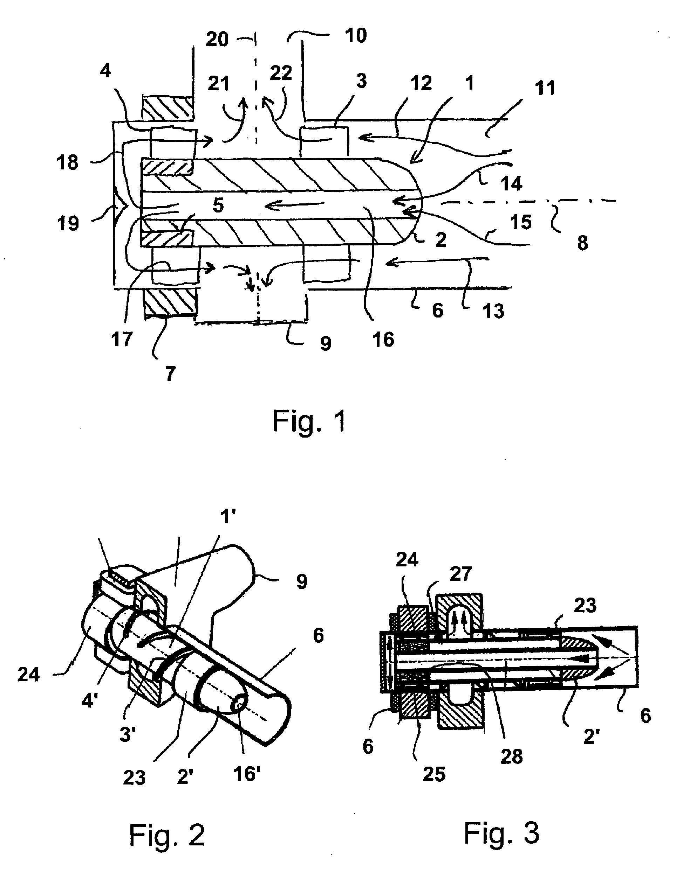

**Figur 1** zeigt schematisch im Längsschnitt eine Rotationspumpe mit einem Rotor 1, der eine Nabe 2 sowie erste Förderelemente 3 und zweite bzw. weitere Förderelemente 4 an seinem Umfang aufweist. Eine Lagerung des Rotors ist nicht im Einzelnen dargestellt, wobei jedoch schematisch eine axiale Magnetlagerung durch einen in die Nabe 2 eingelassenen mitrotierenden Magneten 5 und einen ortsfesten, am Pumpengehäuse 6 außen befestigten Statormagneten 7 angedeutet ist. Weitere Lagerelemente sind nicht dargestellt, auf Beispiele für Lagerungen wird jedoch weiter unten noch genauer eingegangen.

Der Magnet 5 kann auch als Rotormagnet eines elektromotorischen Antriebs ausgebildet sein, wobei dann am Gehäuse eine Statorwicklung vorzusehen ist.

Das Pumpengehäuse 6 ist im Wesentlichen zylindrisch mit einer Rotationssymmetrie um die Achse 8 ausgebildet.

Im Bereich des Rotors 1 ist das Gehäuse 8 von einem sogenannten Spiralgehäuse 9 umgeben, in das das zu fördernde Fluid von dem Rotor 1 aus radial nach außen gefördert wird. Das Spiralgehäuse 9 hat am Umfang in einem begrenzten Bereich einen radialen Auslass 10. In dem dargestellten Beispiel ist in dem Fluidraum 11 des Pumpengehäuses 6 ein Fluid, beispielsweise Blut, vorhanden und wird von dort in den Bereich der Förderelemente 3, 4 eingesaugt. Erste Pfeile 12, 13 zeigen, dass das Fluid zu den ersten Förderelementen 3, die beispielsweise als am Umfang der Rotornabe 2 verteilte Förderschaufeln ausgebildet sein können, eingesaugt wird. Die Förderschaufeln 3 bewirken eine im Wesentlichen axiale Förderung entlang der Rotornabe 2 in Richtung der Pfeile 12, 13.

Zudem weist die Nabe 2 einen Hohlraum 16 auf, der in ihrem Inneren konzentrisch durchlaufend ausgebildet ist und in den hinein das Fluid, wie durch die zweiten Pfeile 14, 15 angedeutet, hineingesaugt wird. Dies wird dadurch bewirkt, dass durch die weiteren Förderelemente 4 eine Förderung des Fluids in Axialrichtung, d.h. parallel zur Längsachse 8 des Rotors, aber gegensinnig zur Förderung der ersten Förderelemente 3, bewirkt wird, wie durch die Pfeile 17, 18 angedeutet. Dadurch wird am Ende des Rotors 2 das Fluid angesaugt, und der Sog setzt sich bis zu dem Fluidraum 11 fort. An dem dem Fluidraum 11 abgewandten Ende des Rotors 1 findet eine Umlenkung des angesaugten Fluids an einem Leit- oder Umlenkelement 19 statt, das als eine Art Prallblech mit kegelförmiger Struktur, beispielsweise auch mit konkav ausgebildeten Flanken, ausgebildet sein kann. Der Fluidstrom wird dort von der axialen Richtung 14, 15 in eine radiale Richtung und weiter um insgesamt etwa 180° zu den Förderelementen 4 umgelenkt.

Im Bereich der durch die Strichelung 20 angedeuteten Trennfläche treffen die beiden axialen Fluidströme aus den Förderelementen 3, 4 axial aufeinander und werden in Radialrichtung, wie durch die Pfeile 21, 22 angedeutet, umgelenkt. Dabei hat die Fluidströmung hier außer der radialen Komponente eine azimutale Komponente, so dass durch das spiralig ausgebildete Gehäuse 9 auch die azimutale Komponente genutzt werden kann, um einen Fluidstrom durch den Auslass 10 radial aus der Pumpe heraus zu fördern.

Somit kann mit der dargestellten Pumpe trotz axialer Ansaugung und zweier Gruppen von gegensinnig axial fördernden Förderelementen 3, 4 ein Förderstrom eines Fluids erzeugt werden, wobei die axialen Schubkräfte auf die Förderelemente sich wenigstens teilweise, idealerweise vollständig, aufheben. Damit kann die Axiallagerung, die schematisch durch die Magnete 5, 7 angedeutet ist, im konstruktiven Aufwand minimiert werden.

**Figur 2** zeigt schematisch in einer dreidimensionalen Ausgestaltung eine ähnliche Rotationspumpe wie Fig. 1 mit gewissen konstruktiven Unterschieden. Es ist ein Teil des Spiralgehäuses 9 durchbrochen dargestellt und ein Rotor 1' mit gegensinnig angelegten Förderelementen 3', 4' sichtbar.

Der Rotor 1' weist einen längs durchgehenden Hohlraum 16' in der Nabe 2' auf. Zudem weist der Rotor 1' sogenannte Stützringe 23, 24 auf, die mit dem Rotor mitrotieren und an der Wand des Pumpengehäuses 6 gelagert sein können, beispielsweise hydrodynamisch. Zwischen den Stützringen 23, 24 und der Rotornabe 2' kann das angesaugte Fluid, also im oben genannten Beispiel Blut, in dem Hauptströmungskanal als Hauptförderstrom frei durchströmen, während ein Nebenstrom zwischen den Stützringen und der Wand des Pumpengehäuses unter Bildung einer stabilisierten, als Gleitschicht wirkenden Flüssigkeitsschicht hindurchströmt, wodurch ein hydrodynamisches Lager gebildet wird.

**Figur 3** zeigt die in Fig. 2 dreidimensional dargestellte Pumpe noch einmal in einem Längsschnitt. Dort sind die Stützringe 23, 24 im Bereich zwischen der Rotornabe 2' und dem Gehäuse 6 zu erkennen. Außerdem ist eine aufwendigere Magnetlagerung mit einer Steuerspule 25, zwei Statormagneten 26, 27 und einem Rotormagneten 28 dargestellt. Diese geregelte Magnetlagerung dient der axialen Positionierung und Lagerung der Rotornabe 2'.

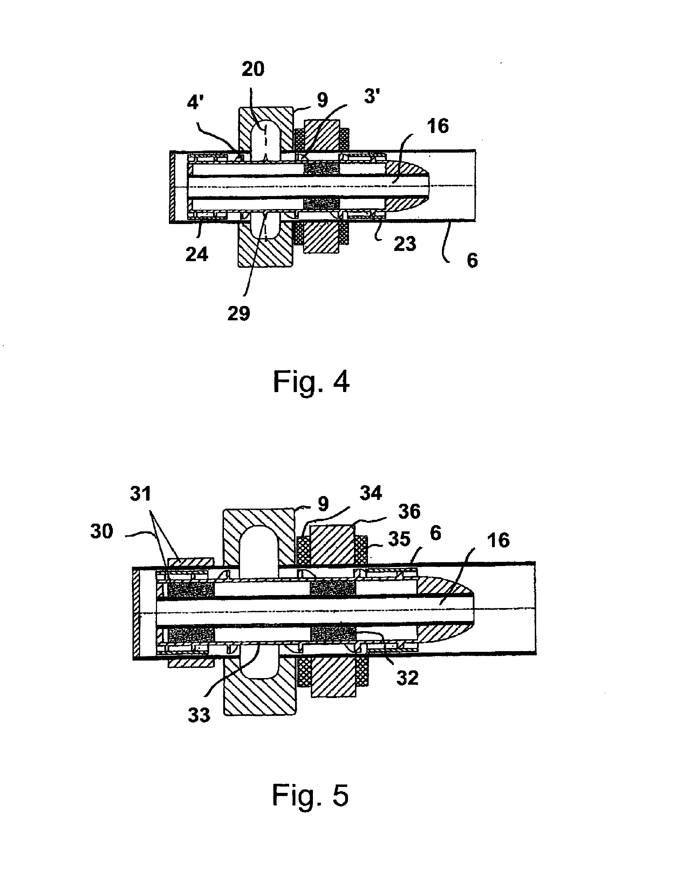

**Figur 4** zeigt eine Rotationspumpe in einem Längsschnitt mit einem hohlen Rotor, der in seinem Inneren einen Kanal 16 für einen Teil des Fluids aufweist, sowie mit ersten Förderelementen 3' und zweiten Förderelementen 4', die am Umfang des Rotors das Fluid in jeweils gegensinnig gerichtete Axialrichtungen fördern. Der Rotor weist im Bereich seiner Enden zwei Stützringe 23, 24 auf, die eine Radiallagerung am Gehäuse 6 bewirken.

Innerhalb des Spiralgehäuses 9 ist im Bereich der Trennlinie 20, wo die beiden Teilfluidströme von den Förderelementen 3', 4' gefördert zusammentreffen, eine Leiteinrichtung 29 in Form einer am Umfang des Rotors umlaufenden kreisringförmigen Flosse dargestellt, die jeweils die axial von beiden Seiten heranströmenden Fluidströme wenigstens teilweise in radiale Richtungen ablenkt.

Die in **Figur 5** schematisch dargestellte Rotationspumpe verzichtet auf eine Leiteinrichtung 29, wodurch die beiden axial aufeinandertreffenden Teilströme im Bereich des Spiralgehäuses 9 gemeinsam radial entlang einer Strömungsgrenzfläche abgelenkt werden. Anhand der Fig. 5 ist speziell eine aufwendigere Magnetlagerung mit einem passiven Magnetlager dargestellt, das einen Magnetring 30 im Rotor und einen Eisenring 31 entsprechend außen am Pumpengehäuse 6 aufweist. Außerdem weist der Rotor einen Magnetring 32 auf, der ebenfalls an dem Rotor befestigt und in seinem Inneren ringförmig zwischen dem zylindrischen Hohlraum 16 und einem die Förderelemente tragenden zylindrischen Außenblech 33 angeordnet ist und der mit einer aktiven Magnetlagereinrichtung mit zwei Ringmagneten 34, 35 und einer Steuerspule 36 zusammenwirkt, mit der die Magnetfeldstärke steuerbar ist.

Zudem kann ein Sensor zur Aufnahme der Axialposition des Rotors vorgesehen sein, der die aktuelle Axialposition des Rotors misst und als Regelgröße in einen Regelprozess einspeist.

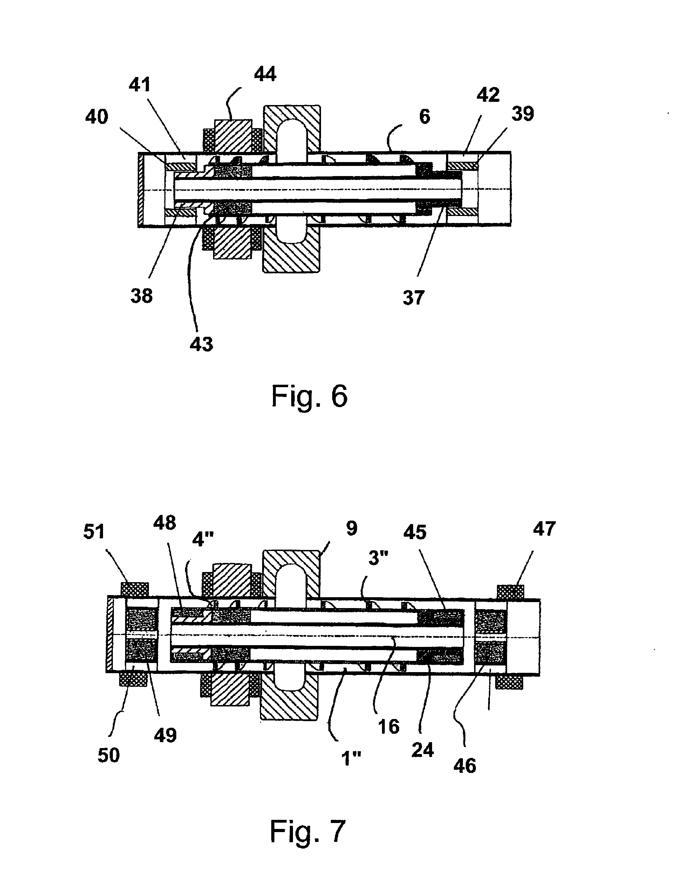

Anhand der **Figur 6** ist eine andere Variante einer Rotationspumpe dargestellt, bei der die Enden 37, 38 eines Rotors in Radiallagerstellen 39, 40 gelagert sind, welche zudem außen zwischen dem Lager und der Gehäusewand des Gehäuses 6 feststehende Leitschaufeln 41, 42 tragen. Die Leitschaufeln sind derart positioniert und geformt, dass sie vor dem Einfließen der Teilfluidströme in den Bereich der Förderelemente eine geeignete Vorausrichtung des Fluidstroms (Vordrall) erzeugen. Der Rotor weist außerdem einen Magnetkörper 43 auf, der mit einem aktiv geregelten Magnetlager 44 zur Axiallagerung zusammenwirkt.

In **Figur 7** ist eine Rotationspumpe mit einem Rotor 1 " dargestellt, der zwei Gruppen von gegensinnig wirkenden Förderelementen 3 " , 4" aufweist, wobei ein Teilfluidstrom durch einen Hohlraum 16 der hohlen Nabe 2 " strömt, bevor er umgelenkt und auf die Förderelemente 4 " geleitet wird.

Der Rotor ist mit zwei aktiven Magnetlagerungen an seinen beiden Enden versehen. Am ersten Ende weist der Rotor einen Magnetring 45 auf, der mit einem feststehenden Magnetring 46 wechselwirkt. Der stehende Magnetring 46 wird zudem durch eine Steuerspule 47 gesteuert.

Am gegenüberliegenden Ende des Rotors ist ebenfalls ein Magnetring 48 im Rotor vorgesehen, der mit einem stationären Magnetring 49 im Gehäuse zusammenwirkt, wobei der Magnetring 49 außen an seinem Umfang stehende Leitschaufeln aufweist und mit einer außen am Gehäuse der Pumpe angeordneten Steuerspule 51 zur Bildung eines geregelten Axiallagers zusammenwirkt.

Die Anordnung weist damit zwei geregelte Axiallager an den Enden des Rotors sowie eine Axialschubkraftkompensation am Rotor durch zwei Gruppen von gegeneinander fördernden Förderelementen auf.

Die Leistung und der Regelungsaufwand der Axiallager sind durch die Gestaltung der Pumpe mit der Kompensation der Axialkräfte wesentlich reduziert.

## Patentansprüche

1. Rotationspumpe mit einem Rotor (1, 1', 1''), der Förderelemente aufweist, welche ein Fluid wenigstens teilweise in Axialrichtung des Rotors fördern,
**dadurch gekennzeichnet,**
**dass** wenigstens ein erstes Förderelement (3, 3', 3'') oder eine erste Gruppe von Förderelementen zur Förderung des Fluids wenigstens teilweise in Axialrichtung in einem ersten Richtungssinn (12, 13) sowie wenigstens ein zweites Förderelement (4, 4', 4'') oder eine zweite Gruppe von Förderelementen zur Förderung des Fluids wenigstens teilweise in Axialrichtung in einem zweiten Richtungssinn (17, 18), der dem ersten Richtungssinn entgegengesetzt ist, vorgesehen ist.

2. Rotationspumpe nach Anspruch 1, **dadurch gekennzeichnet, dass** der durch das erste Förderelement (3, 3', 3'') geförderte erste Fluidstrom und der durch das zweite Förderelement (4, 4', 4 ") geförderte zweite Fluidstrom voneinander separiert sind.

3. Rotationspumpe nach Anspruch 1 oder 2, **dadurch gekennzeichnet, dass** der erste und der zweite Fluidstrom vor der Berührung mit dem ersten und dem zweiten Förderelement voneinander separiert werden.

4. Rotationspumpe nach Anspruch 1, 2 oder 3,
**dadurch gekennzeichnet, dass** der erste und der zweite Fluidstrom nach dem Durchlaufen des ersten und des zweiten Förderelementes (3, 3', 3 " , 4, 4' , 4'') vereinigt werden.

5. Rotationspumpe nach Anspruch 4, **dadurch gekennzeichnet, dass** das erste und das zweite Förderelement den ersten und den zweiten Fluidstrom aufeinander zugerichtet fördern und dass der erste und der zweite Fluidstrom gemeinsam radial nach außen abgelenkt werden.

6. Rotationspumpe nach Anspruch 5, **dadurch gekennzeichnet, dass** das erste und das weitere Förderelement in verschiedenen Abschnitten des Rotors 1, 1', 1'') angeordnet sind und dass axial zwischen diesen Abschnitten ein Spiralgehäuse (9) vorgesehen ist, das den Rotor umgibt.

7. Rotationspumpe nach Anspruch 1 oder einem der folgenden mit einem den Rotor umgebenden Pumpengehäuse (6), **dadurch gekennzeichnet, dass** innerhalb des Pumpengehäuses mehrere separierte Fluidkanäle (16) vorgesehen sind, die einen Fluidraum (11) mit verschiedenen Axialabschnitten, insbesondere den Enden, des Rotors verbinden.

8. Rotationspumpe nach Anspruch 7, **dadurch gekennzeichnet, dass** die Nabe (2, 2', 2'') des Rotors hohl ausgebildet ist und einen Fluidkanal (16) bildet.

9. Rotationspumpe nach Anspruch 7 oder 8, **dadurch gekennzeichnet, dass** die Rotornabe (2, 2', 2'') Radialöffnungen zur Ableitung des Fluids aufweist.

10. Rotationspumpe nach Anspruch 5 oder einem der folgenden, **dadurch gekennzeichnet, dass** der Rotor (1, 1', 1") dort, wo die beiden axialgerichteten Fluidströme aufeinandertreffen, eine Leiteinrichtung (29) aufweist.

11. Rotationspumpe nach Anspruch 1 oder einem der folgenden, **dadurch gekennzeichnet, dass** an feststehenden Teilen der Rotorpumpe feststehende Lenkungseinrichtungen (41, 42) für Fluidströme vorgesehen sind.

12. Rotationspumpe nach Anspruch 11, **dadurch gekennzeichnet, dass** in axialer Verlängerung der Rotornabe (2, 2', 2''), sofern diese hohl ausgebildet ist, eine Pralleinrichtung (19) zur Umlenkung eines Fluidstroms aus einer Axialrichtung in eine oder mehrere Radialrichtungen, insbesondere mit einer zentralen Erhebung, vorgesehen ist.
